# EUROPEAN PATENT APPLICATION

(11) **EP 3 932 573 A1**
(43) Date of publication of application: **05.01.2022**
(21) Application number: 21460029.8
(22) Date of filing: 23.06.2021
(51) Int. Cl.: B08B 7/00

(54) **A DEVICE FOR DISINFECTING CABLES AND WIRES AND A METHOD FOR DISINFECTING CABLES AND WIRES**

(30) Priority: 26.06.2020 PL 43448220
(71) Applicant: Sewertronics Sp. z o.o., 37-114 Bialobrzegi (PL)
(72) Inventor: Kuzniar, Slawomir, 37-100 Lancut (PL); Gómez Jiménez, César, 28200 Madrid (ES)

(57) **Abstract**

A device for disinfecting cables and wires for use in cable coiling/pulling machines which, under their operating conditions, are placed in an environment where the cable/wire is exposed to high exposure to bacteria, viruses and microorganisms, such as pipe ducts, having a body characterised in that all parts (2 and 3) of the body (1) have at least one supporting element (5), and the front (7) and rear (8) wall of the body (1) have openings (9) and (10), and furthermore it has electromagnetic radiation sources (12) inside the body (1).

## Description

The subject of the invention is a device for disinfecting cables and wires for use in cable coiling/pulling machines which, under their operating conditions, are placed in an environment where the cable/wire is exposed to high exposure to bacteria, viruses and microorganisms - such as pipe ducts, and the subject of the invention is a method for disinfecting cables and wires using the device according to the invention.

A cable surface cleaning device with tensioning capability is known from the Chinese utility model description CN210450026U, which includes an air inlet tube, a foam cushion and brackets housed in a cleaning box additionally equipped with a water sleeve constituting a spray head. In this device, the cable is cleaned by means of penetration of a jet of dry air on the surface of the cable, which then travels along the cleaning cushions, the cable being pulled through the device by means of two drive rollers, which additionally maintain the tension of the cable, preventing it from coiling/bending.

Also known from Chinese patent application CN107413885A is a wire cleaning device comprising two cleaning devices, the first of which includes two fixing blocks with layers of wool felt placed between them with a place for the wire to be cleaned, additionally the device includes an air ion cleaning device and a nozzle for spreading and spraying an antioxidant and anticorrosion layer.

The inconvenience of the solutions known so far and at the same time the problem that needs to be solved is the fact that the devices known so far perform cleaning in a mechanical way, with the use of frictional cleaning devices and spray nozzles, which do remove dirt from wires and cables, but this form of cleaning does not allow to get rid of, among others, microorganisms, bacteria or viruses that may be present on these elements, which requires a significantly increased physical and chemical activity on their surfaces.

The purpose of the invention is to develop such a type of device for disinfecting cables and wires, as well as to develop a method for their disinfection, which will be free from the disadvantages known in the state of the art, and which will ensure elimination of the vast majority of bacteria, viruses and microorganisms present on cables/wires and which will be used as equipment in devices known to date. A further purpose of the invention is to develop a method of disinfection which allows disinfecting cables or wires without exposing the user of the device to direct exposure to the disinfectant medium in an enclosed working space.

The nature of a device for disinfecting cables and wires according to the invention is characterised in that all parts of its body have at least one supporting element, while the front and rear walls of the body have openings made therein, and furthermore it has electromagnetic radiation sources inside the body. Advantageously, the supporting elements have a semi-circular bend forming an opening coaxially positioned with respect to the openings of the body walls, and when the electromagnetic radiation sources are placed in each of the body parts. It is also advantageous when the body of the device has a wall with an opening coaxially positioned with respect to the other openings, arranged between the rear wall of the body and the electromagnetic radiation source to form a chamber, and when the chamber has a chemical agent spraying nozzle supplied by a line from a chemical agent container, or when the chamber has an ozone supply outlet, and when, the device according to the invention has elements for mechanical pre-cleaning of a cable or a wire between the electromagnetic radiation source and the front wall of the body. It is also advantageous when the electromagnetic radiation sources are UV LED modules or UV diode modules or UV LED fluorescent lights, and when the UV electromagnetic radiation sources emit radiation in the range of 10-400 nm, and especially when the UV electromagnetic radiation sources emit radiation with a wavelength in the range of 100-280 nm. It is also advantageous if the electromagnetic radiation sources are powered by electrical cables led outside the device through a cable gland.

The essence of a method of disinfecting cables and wires is characterised in that the cable or wire, placed on a device having the ability of winding it up, is pulled through the body of the disinfecting device and is exposed to UV electromagnetic radiation in the range of 10-400 nm. Advantageously, the cable/wire is further exposed to biocides supplied to the body of the disinfecting device or the cable/wire is further exposed to ozone supplied to the body of the disinfecting device. It is also advantageous when the disinfecting device constitutes a device according to the invention.

The advantage of using the device according to the invention is the fact of excellent disinfection of the external surface of cables or wires by means of a device of relatively small dimensions in which the components used in the form of UV diodes/lights allow to eliminate most bacteria and viruses, while the use of the second chamber equipped additionally with a nozzle enabling distribution - supply of chemicals in the form of known disinfecting liquids and equipped with an ozone nozzle allows to multiply the disinfection process increasing the spectrum of application and elimination of particular species of microorganisms requiring elimination. Moreover, the two-chamber design increases the safety of using this device, while the use of spacing elements allowed for centring - placing the cable advantageously in the axis of this device and maintaining its equal distance from individual sources of electromagnetic radiation, which additionally enabled uniform exposure of its external surface from each side.

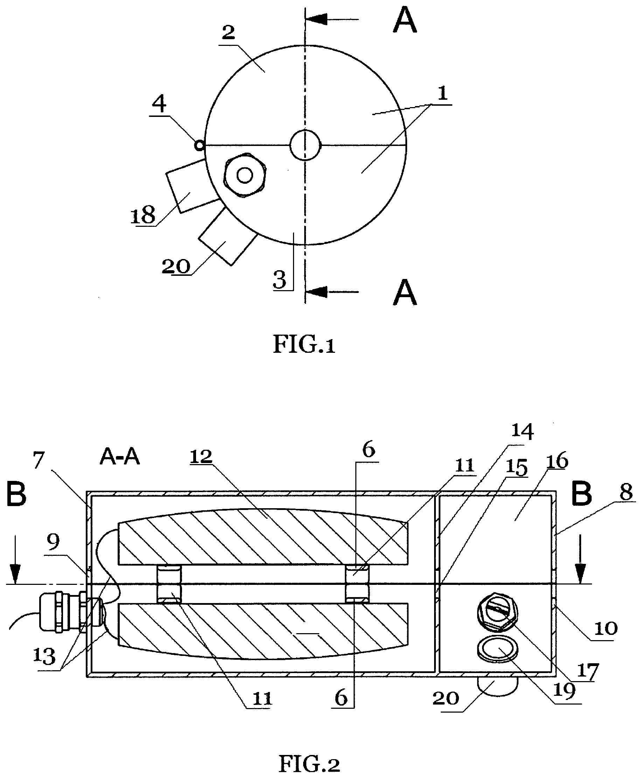

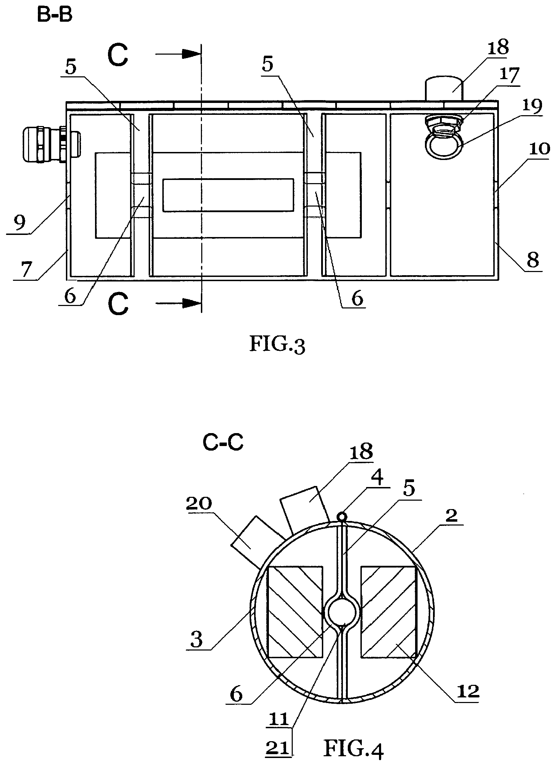

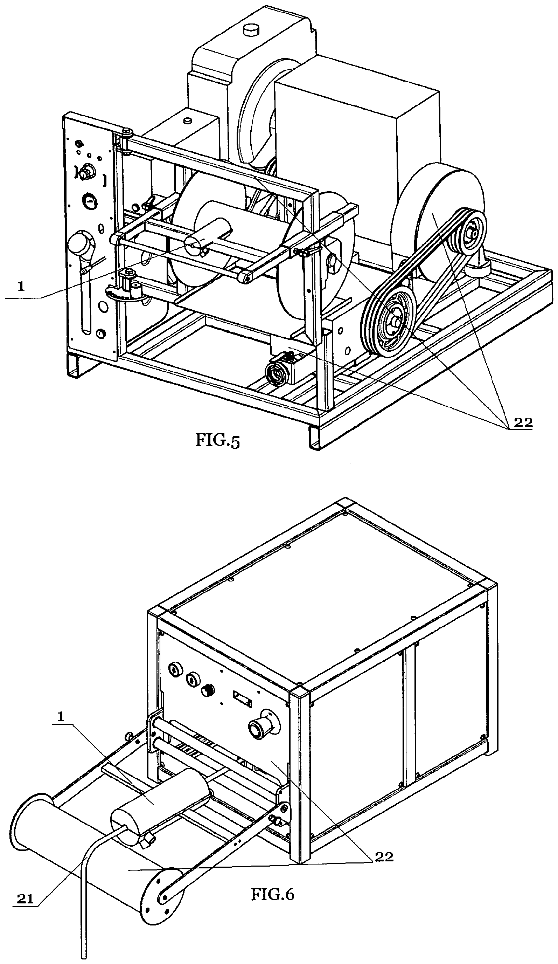

The subject of the solution according to the invention is shown in an exemplary embodiment in Fig. 1-5, in which: Fig. 1 - shows a front view of the disinfecting device in a two-chamber variant with a chemical agent spraying nozzle and an ozone supply line, Fig. 2 - shows a cross-section along line A-A of the same device, Fig. 3 - shows a cross-section along line B-B of the same device, Fig.4 - shows a vertical cross-section along line C-C of the same device same device, Fig.5 - shows a view of the same device in its embodiment placed on a device cleaning sewers with water under high pressure, and Fig. 6 - shows a view of the same device in its embodiment placed on a device for curing a pipeline sleeve with UV light.

### EMBODIMENT 1

A disinfecting device according to the invention consists of a body 1 formed of two identical parts 2 and 3 with U-shaped cross-section, connected to each other by a hinge 4, the open ends of which are connected by two crosswise, longitudinal elements in the form of flat supporting elements 5 with a curved - semi-circular bend 6 in the middle of their length, the inner radius R of which is larger than the radius of the cable intended to be placed in this device. In addition, a front wall 7 and a rear wall 8 of the body 1 have openings 9 and 10 made accordingly, positioned coaxially with respect to openings 11 formed by the semi-circular bends 6 when the device is closed. In addition, inside the body 1, both parts 2 and 3 have longitudinal electromagnetic radiation sources 12 in the form of UVC LED modules positioned between each of the two flat supporting elements 5 and the outer walls of the respective parts 2 and 3, which are connected to a power source, not shown in the figure, by means of electrical cables 13 brought outside the device through a cable gland.

### EMBODIMENT 2

In the second embodiment, the device has a design analogous to the device as in the first embodiment, except that its body 1 has an additional wall 14 with an opening 15 coaxially positioned with respect to the openings 9, 10 and 11 and formed between the rear wall 8 of the body and the end of the electromagnetic radiation source 12, forming an additional chamber 16 inside of which and simultaneously on the surface of the side wall of the part 3 of the body 1 there is placed a nozzle 17 for spraying chemicals in the form of known chemical agents, supplied by a line 18 from a chemical agent container not shown in the figure.

### EMBODIMENT 3

In the third embodiment, the device has a design analogous to the device as in the second embodiment, except that the additional chamber 16, on the side wall 2 of the body 1, has an outlet 19, located next to the nozzle 17, for supplying ozone conveyed to the device via conduits 20 from an external ozone container not shown in the figure.

### EMBODIMENT 4

In the fourth embodiment, the device has a design analogous to the device as in the third embodiment, wherein the chamber 16 has only the outlet 18 of the ozone supply line.

In further embodiments of the device according to the invention, not shown in the figures, the source of the UV electromagnetic radiation is a set of LEDs or LED fluorescent lights, and the two parts 2 and 3 of the body 1 are connected to each other by means of an articulated mechanism or by means of male-and-female connectors, and have one, three or five supporting elements 5 having additionally in their cross-section a semi-circular shape with bends 6 quantitatively selected according to the needs and length of the body 1. Moreover, in further embodiments not shown in the figures, the device according to the invention is provided with additional frictional elements - mechanically pre-cleaning the cable/wire, advantageously in the form of sponges, brushes, felts, which are placed between the electromagnetic radiation source 12 and the front wall 7 of the body 1 of the device, while the supporting element 5 adheres directly to the front wall 7 or to the rear wall 8 of the body 1 in such a way that the bends 6 constitute an extension of the openings 9 and 10.

The UV electromagnetic radiation sources used in the above embodiments of the invention emit radiation in the range of 10-400 nm, advantageously there are used UVC diodes/modules/lights emitting radiation with a wavelength in the range of 100-280 nm.

An example of the implementation of the method according to the invention consists in that the beginning of a cable 21 mounted as standard on a device 22 for cleaning sewers with high-pressure water is placed inside the body 1 in the bends 6 of the flat elements 5 of the device according to the invention described in the embodiments 1-4, whereupon the body 1 is closed and the power supply is switched on, as a result of which the cable pulling mechanism of the device 22 sets the cable in motion, which is pulled through the chamber of the body 1 moving uniformly through the openings 9, 11, 15 and 10 while being simultaneously exposed to the electromagnetic radiation source 12 and/or is subsequently subjected to spraying with chemical agents in the form of known liquid biocides with the nozzle 17 and is subjected to ozone treatment with the conduit 19.

Due to the design of the device and the ease with which the method can be implemented in known devices for handling all types of cables, the device according to the invention can be used in all types of cable and wire winches or winders as an additionally fixed equipment therein, including but not limited to devices adapted for cleaning sewers and devices with UV heads for curing sleeve linings of pipelines.

## Claims

1. A device for disinfecting cables and wires **characterised in that,** all parts (2 and 3) of its body (1) have at least one supporting element (5), while the front wall (7) and rear wall (8) of the body (1) have openings (9 and 10) made therein, and furthermore it has electromagnetic radiation sources (12) inside the body (1).

2. The device as claimed in claim 1, **characterised in that,** the supporting elements have a semi-circular bend (6) forming an opening (11) coaxially positioned with respect to the openings (9 and 10).

3. The device as claimed in claim 1, **characterised in that,** the electromagnetic radiation sources (12) are placed in each parts (2 and 3) of the body (1).

4. The device as claimed in claim 1, **characterised in that,** the body (1) has a wall (14) with an opening (15) coaxially positioned with respect to the openings (9, 10), arranged between the rear wall (8) of the body and the electromagnetic radiation source (12) to form a chamber (16).

5. The device as claimed in claim 4, **characterised in that,** the chamber (16) has a chemical agent spraying nozzle (17) supplied by a line (18) from a chemical agent container.

6. The device as claimed in claim 4, **characterised in that,** the chamber (16) has an ozone supply outlet (19) via a line (20).

7. The device as claimed in claim 1, **characterised in that,** the device has frictional elements for mechanical pre-cleaning of a cable or a wire between the electromagnetic radiation source (12) and the front wall (7) of the body (1).

8. The device as claimed in claim 1 or 4, **characterised in that,** the electromagnetic radiation sources (12) are UV LED modules.

9. The device as claimed in claim 1 or 4, **characterised in that,** the electromagnetic radiation sources (12) are UV diode modules.

10. The device as claimed in claim 1 or 4, **characterised in that,** the electromagnetic radiation sources (12) are UV LED fluorescent lights.

11. The device as claimed in claim 8 or 9 or 10, **characterised in that,** the UV electromagnetic radiation sources emit radiation in the range of 10-400 nm.

12. The device as claimed in claim 8 or 9 or 10, **characterised in that,** the UV electromagnetic radiation sources emit radiation with a wavelength in the range of 100-280 nm.

13. The device as claimed in claim 1 or 8 or 9 or 10, **characterised in that,** the electromagnetic radiation sources (12) are powered by electrical cables (13) led outside the device through a cable gland.

14. A method of disinfecting cables and wires, **characterised in that,** the cable or wire, placed on a device (22) having the ability of winding it up, is pulled through the body (1) of the disinfecting device and is exposed to UV electromagnetic radiation in the range of 10-400 nm.

15. The method of disinfecting as claimed in claim 14, **characterised in that,** the cable/wire is further exposed to biocides supplied to the body (1) of the disinfecting device.

16. The method of disinfecting as claimed in claim 14, **characterised in that,** the cable/wire is further exposed to ozone supplied to the body (1) of the disinfecting device.

17. The method of disinfecting as claimed in claim 14 or 15 or 16, **characterised in that,** the disinfecting device constitutes a device according to claims 1-13.
